# EUROPEAN PATENT SPECIFICATION

(11) **EP 0 321 489 B1**
(45) Date of publication and mention of the grant of the patent: **17.03.1993**
(21) Application number: 87905817.0
(22) Date of filing: 29.07.1987
(51) Int. Cl.: C12Q 1/70, C12Q 1/68, C12N 7/00

(54) **MOLECULAR CLONING AND CLONES OF HUMAN B LYMPHOTROPIC VIRUS (HBLV)**
MOLEKULARE KLONIERUNG UND HUMAN B LYMPHOTROPISCHER VIRUS KLON (HBLV)
CLONAGE MOLECULAIRE ET CLONES DU VIRUS LYMPHOTROPE B HUMAIN (HBLV)

(30) Priority: 12.08.1986 US 895857
(43) Date of publication of application: 28.06.1989
(73) Proprietor: THE UNITED STATES OF AMERICA as represented by the Secretary United States Department of Commerce, Springfield, Virginia 22161 (US)
(72) Inventor: JOSEPHS, Steven, Francis, Rockville, MD 20851 (US); GALLO, Robert, C., Bethesda, MD 20817 (US); WONG-STAAL, Flossie, Yeeching, Potomac, MD 20854 (US); SALAHUDDIN, Syed, Zaki, Potomac, MD 20854 (US)
(74) Representative: Lamb, John Baxter
(86) International application number: US8701817
(87) International publication number: WO8801305

(56) References cited:
- WO-A-84/04327
- WO-A-86/02930
- US-A- 4 237 224
- SCIENCE, vol. 234, 31st October 1986, pages 601-603; S.F. JOSEPHS et al.: "Genomic analysis of the human B-lymphotropic virus (HBLV)"
- DAVIES et al., MICROBIOLOGY, 3rd., Chapter 55, pages 1061-1076, issued 1980 (Harper & Row, Publishers, Hagerstown);
- MANIATIS et al., MOLECULAR CLONING A LABORATORY MANUAL, pages 98-106 and 270-307, issued 1982 (Cold Spring Harbor Laboratory, Cold Spring Harbor, New York);

## Description

A new DNA virus, designated Human B Lymphotropic Virus (HBLV), has been isolated from the blood leukocytes of patients with lymphoproliferative disorders. While the virus belongs morphologically to the Herpes family of viruses, HBLV, as shown below, this virus has not been previously characterized. HBLV is associated with some malignancies in AIDS and non-AIDS patients, but is distinctly different than Human T-cell Lymphotropic Virus Type III (HTLV-III), the causative agent of AIDS. HBLV contains a large double stranded DNA genome, and selectively infects B cells; HTLV-III, on the other hand, contains a single stranded RNA genome, and selectively infects and is cytolytic for T cells.

The nucleocapsid of the HBLV virus is of icosahedral symmetry with 162 capsomeres, and is enveloped in a lipid membrane. The outer surface of the viral envelope is covered with short spikes. The diameter of the enveloped virion is approximately 180 nm; the nucleocapsid is approximately 100 nm in diameter. The space between the capsid and the envelope, 35-40 nm, is filled with amorphous material. The nucleoprotein core or nucleoid is approximately 65 nm in diameter, and is occasionally rod-shaped or asymmetric.

Infection of primary cells or of cord blood cells produces characteristic large cells 4-10 days after infection. These cells are 2-4 times the diameter of small leukocytes, and exhibit cytopathogenic and cytolytic changes after about one week in culture. The nuclei of these cells is often highly convoluted, containing mainly euchromatic chromatin and nucleoli without remarkable features. Large numbers of virions are released by most infected cells.

### GENERAL DESCRIPTION OF THE INVENTION

The present invention is the production of a molecular clone of Human B Lymphotropic Virus (HBLV) and the use of that clone in diagnostic procedures.

A new Human B Lymphotropic Virus (HBLV) has been isolated from the peripheral blood leukocytes of six individuals: three Human T-cell leukemia virus type III (HTLV-III) seropositive patients with Acquired Immune Deficiency Syndrome (AIDS)-related lymphoma, two HTLV-III seropositive patients with angio-immunoblastic lymphadenopathy, and one patient with acute lymphoblastic leukemia. All six isolates are closely related by antigenic and molecular analysis, and sera from all six virus-positive patients react immunologically with each virus isolate. In contrast, only four sera from more than 200 randomly selected healthy donors were seropositive. HBLV contains a large double stranded DNA genome and is morphologically similar to some members of the Herpes virus group. HBLV selectively infects freshly isolated human B cells, where it induces the appearance of characteristic large, refractile mononucleated or binucleated cells containing nuclear and cytoplasmic inclusion bodies. However, HBLV is distinguishable from all the known human and sub-human primate Herpesviruses by host range, biological effect on infected cells, and by lack of antigenic or genomic relatedness.

In one of the preferred embodiments of the present invention, molecular clone pZVH14 is used in the detection of early virus infection of umbilical cord blood lymphocytes and spleen cells by in situ hybridization in culture.

It is believed that the present invention is capable of introducing genes into target cells of the virus as a recombinant DNA virus vector system.

The ultrastructural characteristics of the HBLV virus, as well as its morphogenesis, place the virus in the family of Herpesviruses -- with similarities to and differences from any known members of the family. Immune electron microscopic studies show that patients from whom the virus has been isolated make highly specific antibodies to both the viral envelope and to internal components of the virus. Immunological, molecular, biological, and host range studies indicate that the HBLV virus has not been previously described.

Cultures of mononuclear cells from infected blood samples develop significant numbers of characteristic large cells 4-10 days after culture with primary cells or cord blood cells. Electron microscopy analysis shows that HBLV virus particles are present in large cells but absent in small lymphocytes. The infected cells are 2-4 times the diameter of small lymphocytes and do not show any initial obvious cytopathic changes. After one week in culture, however, cytopathic and cytolytic changes are readily observable. Specifically, the nuclei of infected cells are often highly convoluted; chromatin is mainly euchromatic and contain nucleoli without remarkable features. The cytoplasm displayed fairly large Golgi apparatus, vesicles of different sizes, prominent arrays of rough endoplasmic reticulum, and abundant mitochrondria. The general appearance of these cells is that of highly polymorphic proliferating blasts of lymphoid origin.

Specific immunolabeling of extracellular virus occurs at the ultrastructural level using pre-absorbed patient's serum and an antiserum against human gamma globulin raised in goats, and labeled either with ferritin or with peroxidase. Large numbers of virions are released by most infected cells, and appear in tight clusters at the surface of the cells. Virtually all of the virions are labeled at their periphery. In some instances, the label penetrates into the virion, indicating that the envelopes of some of the virions are not intact and that some of the patient's serum contains antibodies to internal components of the virus as well as to the viral envelope.

The HLBV virus of the present invention is propagated by infecting human cord blood cells with HBLV, as is described in more detail in the Specific Disclosure.

### STATEMENT OF DEPOSIT

The subject matter of this invention, molecular clone pZVH14, has been deposited in the American Type Culture Collection in Rockville, Maryland, under ATCC No. 40247, and after allowance of this application, will be maintained for a term of thirty (30) years or five (5) years after the last request for such deposit or for the effective life of the patent, whichever is longest. The deposit will be replaced if the culture mutates or becomes nonviable during the term of the deposit.

### DESCRIPTION OF THE FIGURE

The Figure is the Southern Blot analysis of Human B Lymphotropic Virus genomic DNA.

### UTILITY STATEMENT

The molecular clone of the present invention is capable of in situ hybridization with host cells infected with HBLV. This clone, therefore, is useful as a diagnostic probe for HBLV-infected cells, as well as for the detection of viral antigens or antibodies in blood samples (using an immunofluorescence assay or any other assay for antigens or antibodies which uses viral nucleic acids). Molecular clone pZVH14 is also used in the detection of early virus infection of umbilical cord blood lymphocytes and spleen cells by in situ hybridization in culture.

The present invention is also capable of introducing genes into target cells of the virus as a recombinant DNA virus vector system.

### SPECIFIC DESCRIPTION OF THE INVENTION

In general, one method of cloning the Human B Lymphotropic Virus (HBLV) genome involves isolating unintegrated viral DNA after infection of primary cells or cord blood cells with the HBLV virus. The unintegrated viral DNA is then cloned in a lambda phage library and screened with viral cDNA.

Infected primary cells and cultured peripheral cord blood cells produce HBLV virus and serve as the principal producer for immunological assays used to detect virus specific antigens and antibodies in human sera. Cultures of infected cells are grown and harvested, followed by extraction of low molecular weight DNA from newly infected cells. This produces unintegrated viral DNA. A cDNA library is formed using HBLV cDNA. This cDNA is then used as a probe for assaying unintegrated viral DNA. Unintegrated linear DNA (provirus DNA) is then obtained, containing the HBLV genome of the present invention. This DNA is then digested in a suitable plasmid to form clone pZVH14.

Two elements of the above process are well known recombinant DNA procedures: the DNA library and the cDNA probe. The library is formed by taking the total DNA from the infected primary or peripheral blood cells, cutting the DNA into fragments with a suitable restriction enzyme, hybridizing the fragments to a radiolabeled cDNA probe, joining the fragments to plasmid vectors, and then introducing the recombinant DNA into a suitable host.

The cDNA probe is an HBLV cDNA probe made from double-stranded HBLV mRNA. A short oligo-dT chain is hybridized to the poly-A tail of the mRNA strand. The oligo-dT segment serves as a primer for the action of reverse transcriptase, which uses the mRNA as a template for the synthesis of a complementary DNA strand. The resulting cDNA ends in a hairpin loop. Once the mRNA strand is degraded by treatment with NaOH, the hairpin loop becomes a primer for DNA polymerase I, which completes the paired DNA strand. The loop is then cleaved by S1 nuclease to produce a double-stranded cDNA molecule. Linkers are then added to the double-stranded cDNA by using DNA ligase. The linkers are cut open with a restriction enzyme and the cDNA is inserted into a suitable plasmid cleaved with the same enzyme; the result is a cDNA-containing recombinant plasmid.

As shown in Example 5, the molecular clone pZVH14 of the HBLV genome is useful as a template for radiolabeled RNA using T7 RNA polymerase, ³⁵S-labeled dGTP, and unlabeled ribotriphosphates.

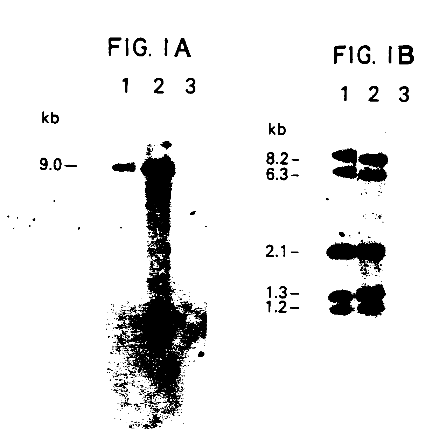

In the preferred embodiment of the present invention, supernatant fluid from HBLV infected umbilical cord blood cells is layered onto 20% glycerol cushions and pelleted by centrifuging at 25,000 rpm for 3 hr. in a Beckman SW41 rotor at 4°C. The pellets are suspended up in TNE buffer (10 mM, Tris-HCl, pH 9, 100 mM NaCl:1 mM EDTA), and extracted with PCI9 (Phenol:Chloroform:Isoamyl alcohol: 50 mM Tris-Cl, pH 9:: 100:100:1:10 :: v:v:v:v) followed Chloroform:Isoamyl alcohol (24:1::v:v). Enriched viral DNA is precipitated by adding 2 volumes of 95% ethanol. DNA is digested with HindIII and cloned into the Bluescrib® vector (commercially available from Vector Cloning Systems, CA). Several clones obtained after screening with labeled, enriched DNA were examined for specificity of hybridization to infected human umbilical cord blood cell DNA and by in situ hybridization to infected cells. Specific hybridization of HBLV clone pZVH14 to DNA from pelleted virus digested with HindIII (Fig. 1, panel A) and EcoRI (Fig. 1, panel B). Extracellular virus is shown in lane 1, virus infected human umbilical cord blood cells in lane 2, and negative control DNA isolated from the skin of an AIDS patient in lane 3. Clone ZVH14 scored positive in these essays and did not hybridize to uninfected controls. The infected cell DNA shown in lane 2 is isolated after several rounds of cell free virus transmission in human umbilical cord blood cells.

### EXAMPLES

Example 1. Several DNA clones obtained from nucleic acids extracted from purified virus obtained as described above, were examined for specificity and for comparison with other DNA viruses. One HBLV clone designated pZVH14, which contained a 9.0 Kb HindIII fragment, was used for these studies. Southern blot analysis showed the presence of viral specific DNA in HindIII and EcoRI digests of DNA from both purified virus and HBLV-infected human cord blood cells. In situ hybridization experiments with the same probe also confirmed that these sequences were confined to infected cells.
Example 2. Human B Lymphotropic Virus clone pZVH14 has been restriction enzyme mapped, as follows: 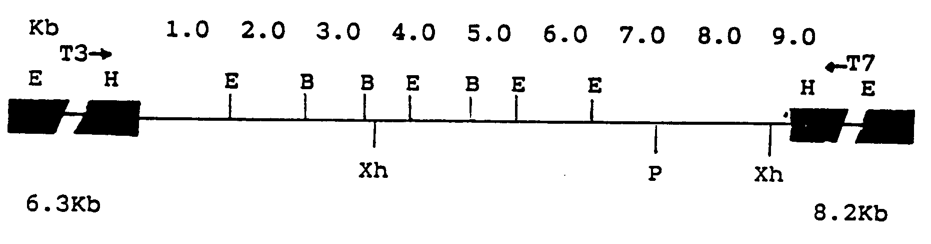
Kb 1.0 2.0 3.0 4.0 5.0 6.0 7.0 8.0 9.0
T3→
←T7
E H E B B E B E E H E
Xh P Xh
6.3Kb 8.2Kb
wherein B=BamHI; E=EcoRI; Xh=XhoI; H=HindIII; P=PstI, and 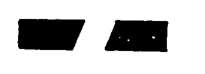
indicates viral fragments detected with pZVH14 insert in infected cells and viral DNA preparations using EcoRI.
Example 3. Molecular probes specific for HSV-1, CMV, and EBV (Herpes Simplex Virus type 1, cytomegalovirus, and Epstein-Barr virus, respectively) were used for comparisons with HBLV. While each individual viral probe specifically hybridized to its homologous nucleic acids, HBLV was clearly distinct from these transforming human DNA viruses. Furthermore, the size of the HBLV genome was shown to contain a minimum complexity of 110kb-pair as determined by analysis of sucrose gradient purified viral DNA. This genome size, as well as other features, also distinguishes HBLV from DNA viruses of the adenovirus, polyomavirus, papovavirus, and papillomavirus groups.

Despite morphological and other properties similar to some of the herpesviruses, HBLV appears to be a new human DNA virus. It is distinguishable from other viruses by biological properties and by a lack of immunological and genomic homology. HBLV is highly lytic in vitro, as are CMV, HSV, herpesvirus saimiri (HVS) and herpesvirus atteles (HVA), but has a narrower host range than these viruses or EBV, being limited to a subset of B-cells.
Example 4. Umbilical cord blood lymphocytes were co-cultured with AIDS patient's blood cells. Characteristic large refractile cells appeared in the cord blood cultures. After six days in culture, the cells were pelleted and the supernatants were layered over 20% glycerol and spun in the centrifuge (Beckman SW41 or SW28) for 3 hours at 25,000-30,000 rpm. The pellets contained cell debris and virus particles. Phenol extraction of the pellet produced large amounts of viral nucleic acid. A double-stranded DNA clone, pZVH14, obtained from this nucleic acid preparation was used for specific hybridization detection of the virus in Southern Blot experiments and in in situ hybridization experiments.
Example 5. In situ hybridization of HBLV-infected human cord blood cells. Experiments were performed utilizing ³⁵S-labeled RNA probes as described in the Specific Disclosure. Clone pZVH14 of the HBLV genome was used as a template for radiolabeled RNA using T7 RNA polymerase, ³⁵S-labeled dGTP, and unlabeled ribotriphosphates. Less than one grain per cell was observed in uninfected negative control cultures. Large refractile cells characteristic of the infected cultures were heavily labeled, indicating the expression of abundant viral messages.

## Claims

1. A Human B Lymphotropic Virus, (HBLV), wherein the virus is encoded from a HBLV gene cloned in a molecular clone, pZVH14, having ATCC accession number 40247.

## Patentansprüche

1. Human-B-lymphotropischer Virus, (HBLV), bei welchem der Virus von einem HBLV-Gen, das in einem molekularen Klon, pZVH14, kloniert ist, der die ATCC-Zugangsnummer 40247 besitzt, codiert ist.

## Revendications

1. Virus lymphotrope B human (HBLV), dans lequel le virus est codé comme étant un gène HBLV cloné dans un clone moléculaire, pZVH14, ayant le numéro 40247 d'accession auprès de l'ATCC.
